(19) Europäisches Patentamt
European Patent Office
Office européen des brevets

(11) Veröffentlichungsnummer: **0 016 725**
**B1**

(12) # EUROPÄISCHE PATENTSCHRIFT

(45) Veröffentlichungstag der Patentschrift: **23.03.83**

(51) Int. Cl.[3]: **C 07 D 501/00,** C 07 D 501/04

(21) Anmeldenummer: **80810058.0**

(22) Anmeldetag: **18.02.80**

(54) **Verfahren zur Herstellung von 3-Hydroxy-cepham-Verbindungen.**

(30) Priorität: **23.02.79 CH 1844/79**

(43) Veröffentlichungstag der Anmeldung: **01.10.80 Patentblatt 80/20**

(45) Bekanntmachung des Hinweises auf die Patenterteilung: **23.03.83 Patentblatt 83/12**

(84) Benannte Vertragsstaaten: **BE CH DE FR GB IT NL**

(56) Entgegenhaltungen:
**CH-A-0 585 756**
**DE-A-2 331 078**

(73) Patentinhaber: **CIBA-GEIGY AG, Patentabteilung Postfach, CH-4002 Basel (CH)**

(72) Erfinder: **Scartazzini, Riccardo, Dr., Conrad Ferdinand Meyer-Strasse 38, CH-4059 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 016 725 B1

BUNDESDRUCKEREI BERLIN

# Verfahren zur Herstellung von 3-Hydroxy-cepham-Verbindungen

Die Erfindung betrifft ein neues Verfahren zur Herstellung von Cephamverbindungen, insbesondere von 7β-substituierten-3-Hydroxycepham-4-carbonsäureverbindungen, die wertvolle Zwischenprodukte zur Herstellung von antibiotisch wirksamen 7β-substituierten-3-Cephem-4-carbonsäureverbindungen sind. Die Erfindung betrifft insbesondere ein Verfahren zur Herstellung von 7β-substituierten-3-Hydroxycepham-4-carbonsäureverbindungen aus 7β-substituierten-3-Hydroxycephem-4-carbonsäureverbindungen durch Reduktion mittels komplexer Borhydride in Gegenwart einer unsubstituierten oder durch Halogen substituierten $C_1-C_7$-Niederalkancarbonsäure.

Die Herstellung von 7β-substituierten-3-Hydroxycepham-4-carbonsäureverbindungen aus 7β-substituierten-3-Hydroxy-3-cephem-4-carbonsäureverbindungen, bzw. aus den dazu tautomeren 7β-substituierten-3-Oxocepham-4-carbonsäureverbindungen, durch Reduktion mittels katalytisch aktiviertem Wasserstoff, metallischen Reduktionsmitteln (nascierendem Wasserstoff) und mit Hydrid-Reduktionsmitteln ist bereits in der Deutschen Offenlegungsschrift 2 331 078 beschrieben worden. Spezifisch wurde die Verwendung einer wäßrigen Lösung von Natriumborhydrid in Methanol beschrieben.

Die mit den beschriebenen Reduktionsmitteln erhaltenen Ergebnisse stellten sich, insbesondere bezüglich der Ausbeuten, als unbefriedigend heraus. Es bestand demnach ein Bedürfnis nach einem wirtschaftlich günstigeren Verfahren.

Überraschenderweise wurde gefunden, daß bei der Reduktion der genannten 3-Hydroxy-3-cephem-4-carbonsäureverbindungen mit einem komplexen Borhydrid wesentlich höhere Ausbeuten an entsprechenden 3-Hydroxycephamverbindungen und auch reinere Produkte erhalten werden, wenn man die Reaktion in Gegenwart unsubstituierter oder durch Halogen substituierter $C_1-C_7$-Niederalkancarbonsäuren durchführt.

Gegenstand der vorliegenden Erfindung ist ein Verfahren zur Herstellung von 7β-substituierten-3-Hydroxycepham-4-carbonsäureverbindungen der Formel

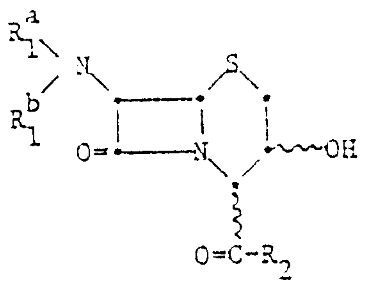

(I)

worin $R_1^a$ Wasserstoff oder eine Aminoschutzgruppe $R_1^A$ bedeutet und $R_1^b$ Wasserstoff oder einen Acylrest Ac darstellt, oder worin $R_1^a$ und $R_1^b$ zusammen eine bivalente Aminoschutzgruppe bilden und $R_2$ einen zusammen mit der Carbonylgruppierung $-C(=O)-$ eine geschützte Carboxylgruppe bildenden Rest bedeutet, von 1-Oxiden davon, sowie Salzen von solchen Verbindungen mit salzbildenden Gruppen, aus einer 7β-substituierten-3-Hydroxy-3-cephem-4-carbonsäureverbindung der Formel

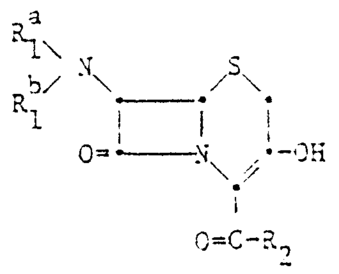

(II)

aus einem 1-Oxid oder einen Salz davon, und einem komplexen Borhydrid, dadurch gekennzeichnet, daß man die Reduktion in Gegenwart einer unsubstituierten oder durch Halogen substituierten $C_1-C_7$-Niederalkancarbonsäure durchführt.

In den Verbindungen der Formel I befinden sich die 3-Hydroxy- und die geschützte 4-Carboxygruppe in α- oder β-Stellung, wobei beide Gruppen bevorzugt in α-Stellungen stehen, da sie bei der Reduktion bevorzugt in diese Stellungen dirigiert werden.

Die Ausgangsverbindungen der Formel II können auch in der tautomeren 3-Oxocephamform vorliegen.

In der nachfolgenden Beschreibung der Erfindung bedeutet der Ausdruck »Nieder« in Gruppen wie

Niederalkyl, Niederalkylen, Niederalkoxy, Niederalkanoyl und dergleichen, daß die entsprechenden Gruppen, sofern nicht ausdrücklich anders definiert, bis zu 7, bevorzugt bis zu 4 C-Atome enthalten.

Die in Verbindungen der Formel I oder II vorhandenen funktionellen Gruppen, insbesondere Carboxyl-, Amino-, Hydroxy-, Hydroxyimino- und Sulfogruppen, sind gegebenenfalls durch Schutzgruppen geschützt, die in der Penicilin-, Cephalosporin- und Peptidchemie verwendet werden.

Solche Schutzgruppen sind leicht, daß heißt ohne daß unerwünschte Nebenreaktionen stattfinden, beispielsweise solvolytisch, reduktiv, photolytisch oder auch unter physiologischen Bedingungen, abspaltbar.

Schutzgruppen dieser Art, sowie ihre Abspaltung sind beispielsweise beschrieben in »Protective Groups in Organic Chemistry«, Plenum Press, London, New York, 1973, ferner in »The Peptides«, Vol. I, Schröder and Lübke, Academic Press, London, New York, 1965, sowie in Houben-Weyl, Methoden der organischen Chemie, 4. Aufl., Bd. 15/1, Georg Thieme Verlag, Stuttgart, 1974.

In den Ausgangsprodukten der Formel II und dem Endprodukt der Formel I haben die Symbole $R_1^A$, $R_1^b$, Ac und $R_2$ beispielsweise die folgenden Bedeutungen:

Eine Aminoschutzgruppe $R_1^A$ ist eine durch Wasserstoff ersetzbare Gruppe, in erster Linie eine Acylgruppe Ac, ferner eine Triarylmethylgruppe, sowie eine organische Silyl- oder Stannylgruppe.

Eine Acylgruppe Ac, die auch den Rest $R_1^b$ bedeuten kann, ist in erster Linie der Acylrest einer organischen Carbonsäure, vorzugsweise mit bis zu 18, und in erster Linie mit bis zu 10, Kohlenstoffatomen, insbesondere der Acylrest einer gegebenenfalls substituierten aliphatischen, cycloaliphatischen, cycloaliphatisch-aliphatischen, aromatischen, araliphatischen, heterocyclischen oder heterocyclisch-aliphatischen Carbonsäure und steht insbesondere für einen in einem natürlich vorkommenden oder in einem bio-, halb- oder totalsynstetisch herstellbaren, vorzugsweise pharmakologisch wirksamen N-Acylderivat einer 6-Amino-penam-3-carbonsäure- oder 7-Amino-3-cephem-4-carbonsäureverbindung enthaltenen Acylrest einer organischen Carbonsäure, vorzugsweise mit bis zu 18, und in erster Linie mit bis zu 10, Kohlenstoffatomen.

Ein solcher Acylrest Ac ist in erster Linie eine Gruppe der Formel

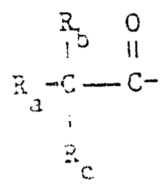

(IA)

worin (1) $R_a$ einen gegebenenfalls substituierten carbocyclischen Arylrest, z. B. entsprechendes Phenyl, einen gegebenenfalls substituierten, vorzugsweise ungesättigten cycloaliphatischen Kohlenwasserstoffrest, z. B. entsprechendes Cyclohexadienyl oder Cyclohexenyl, oder einen gegebenenfalls substituierten heterocyclischen Arylrest, z. B. entsprechendes Thienyl, Furyl oder Thiazolyl, $R_b$ Wasserstoff und $R_c$ Wasserstoff oder gegebenenfalls substituiertes, insbesondere geschütztes Hydroxy, Amino, Carboxyl oder Sulfo darstellen, oder worin (2) $R_a$ gegebenenfalls geschütztes $\omega$-Amino-$\omega$-carboxyniederalkyl, z. B. -propyl, Cyan, veräthertes Hydroxy oder Mercapto, wie gegebenenfalls substituiertes Phenyloxy, Phenylthio oder Pyridylthio, oder einen gegebenenfalls substuierten, über ein Ringstickstoffatom verknüpften, ungesättigten heterocyclischen Rest, z. B. entsprechendes Tetrazoyl, und $R_b$ und $R_c$ Wasserstoff bedeuten, oder worin (3) $R_a$ einen gegebenenfalls substituierten carbocyclischen Arylrest, z. B. entsprechendes Phenyl, oder einen gegebenenfalls substituierten heterocyclischen Arylrest, z. B. entsprechendes Thienyl, Furyl oder Thiazolyl, und $R_b$ und $R_c$ zusammen vorzugsweise O-substituiertes Hydroxyimino in der syn-Konfiguration bedeuten.

Cyclohexadienyl ist insbesondere 1,4-Cyclohexadienyl, während Cyclohexenyl in erster Linie 1-Cyclohexenyl ist.

Thienyl ist vorzugsweise 2-, ferner 3-Thienyl, Furyl bedeutet insbesondere 2-Furyl, Thiazolyl ist insbesondere 4-Thiazolyl, während Pyridylthio z. B. 4-Pyridylthio, und Tetrazolyl z. B. 1-Tetrazolyl darstellen.

Substituenten einer Phenyl- oder Phenyloxygruppe $R_a$ können in irgendeiner Stellung vorhanden sein und sind u. a. aliphatische Kohlenwasserstoffreste, wie gegebenenfalls substituiertes Niederalkyl, z. B. geschütztes Aminomethyl, gegebenenfalls funktionell abgewandeltes, wie veräthertes oder verestertes Hydroxy oder gegebenenfalls substituiertes, insbesondere geschütztes Amino, wie Acylamino, oder Nitro, das z. B. in der Phenyloxygruppe in 2-Stellung sein kann.

Substituenten einer Cyclohexadienyl- oder Cyclohexanylgruppe, sowie einer Thienyl- oder Furylgruppe $R_a$ sind z. B. gegebenenfalls substituiertes Niederalkyl, wie gegebenenfalls substituiertes, z. B. geschütztes Aminomethyl, wobei sich ein solcher Substituent, insbesondere gegebenenfalls geschütztes Aminomethyl, in erster Linie in 2-Stellung eines 1,4-Cyclohexadienyl- oder 1-Cyclohexenylreste oder in 5-Stellung eines 2-Thienyl- oder 2-Furylrestes befindet. Substituiertes Thiazolyl ist insbesondere 2-Amino-4-thiazolyl, worin die Aminogruppe gegebenenfalls geschützt oder durch Niederalkyl, insbesondere $C_1-C_4$-Niederalkyl, z. B. Methyl, Äthyl, Propyl, Isopropyl, Butyl oder

tert.-Butyl, substituiert ist.

Gegebenenfalls geschütztes Aminomethyl ist in erster Linie gegebenenfalls durch Niederalkyl substituiertes Aminomethyl, z. B. Methylaminomethyl, wobei Amino gegebenenfalls geschützt ist, während veräthertes Hydroxy z. B. Niederalkoxy, wie Methoxy, und verestertes Hydroxy z. B. Niederalkanoyloxy, wie Acetyloxy, Aroyloxy, z. B. Benzoyloxy, Carbamoyloxy oder Halogen, z. B. Chlor, und gegebenenfalls substituiertes Amino z. B. durch Niederalkyl substituiertes Amino, z. B. Methylamino, oder Niederalkylsulfonylamino, z. B. Methylsulfonylamino, sein kann.

Geschützte Hydroxy-, Amino-, Carboxyl- oder Sulfogruppen in Acylresten der Formel IA sind solche, die in der Penicillin- und Cephalosporinchemie üblich sind, und die leicht in freie Hydroxy-, Amino-, Carboxyl- oder Sulfogruppen überführt werden können, ohne daß dabei das Cephemgerüst zerstört wird oder andere unerwünschte Nebenreaktionen stattfinden.

Aminogruppen können z. B. durch Acylreste geschützt sein, wobei ein Acylrest in erster Linie ein durch Reduktion, z. B. beim Behandeln mit einem chemischen Reduktionsmittel oder mit katalytisch aktiviertem Wasserstoff, oder durch Solvolyse, z. B. durch Behandeln mit einer geeigneten Säure, ferner auch mittels Bestrahlen, abspaltbarer Acylrest eines Halbesters der Kohlensäure ist, wie ein, vorzugsweise am ersten Kohlenstoffatom der veresternden Gruppe mehrfach verzweigter und/oder durch Aryl, z. B. gegebenenfalls, wie durch Niederalkoxy, z. B. Methoxy, und/oder Nitro substituiertes Phenyl oder Biphenyl oder durch Arylcarbonyl, insbesondere Benzoyl, substituierter Niederalkoxycarbonylrest, z. B. tert.-Butyloxycarbonyl, tert.-Pentyloxycarbonyl, Diphenylmethoxycarbonyl, 1-(4-Biphenylyl)-1-methyl-äthoxycarbonyl, Benzyloxycarbonyl, 4-Nitrobenzyloxycarbonyl, 2-Nitro-4,5-dimethoxy-benzyloxycarbonyl oder Phenacyloxycarbonyl, oder am zweiten Kohlenstoffatom der veresternden Gruppe durch Halogen substituierter Niederalkoxycarbonylrest, z. B. 2,2,2-Trichloräthoxycarbonyl oder 2-Jodäthoxycarbonyl (oder ein in letzteren überführbarer Rest, wie 2-Chlor- oder 2-Bromäthoxycarbonyl), ferner polycyclisches Cycloalkoxycarbonyl, z. B. Adamantyloxycarbonyl.

Eine Aminogruppe kann ferner durch einen Arylmethyl-, wie Polyarylmethylrest, z. B. durch Trityl, eine 2-Carbonyl-vinyl Gruppierung, wie eine 1-Niederalkoxycarbonyl-1-propen-2-ylgruppe, z. B. 1-Methoxycarbonyl-1-propen-2-yl, eine Arylthio- oder Arylniederalkylthiogruppe, z. B. 2-Nitrophenylthio oder Pentachlorphenylthio, ferner Tritylthio, oder eine Arylsulfonylgruppe, ferner durch eine organische Silyl- oder Stannylgruppe, wie eine durch Niederalkyl, Halogen-niederalkyl, Cycloalkyl, Phenyl oder Phenylniederalkyl, oder gegebenenfalls abgewandelte funktionelle Gruppen, wie Niederalkoxy, z. B. Methoxy, oder Halogen, z. B. Chlor, substituierte Silyl- oder Stannylgruppe, in erster Linie Triniederalkylsilyl, z. B. Trimethylsilyl, Halogen-niederalkoxy-niederalkylsilyl, z. B. Chlormethoxymethylsilyl, oder auch Triniederalkylstannyl, z. B.Tri-n-butylstannyl, geschützt sein.

Hydroxyschutzgruppen sind z. B. Acylreste, insbesondere einer der im Zusammenhang mit einer geschützten Aminogruppe genannten Acylreste von Kohlensäurehalbestern, oder organischen Silyl- oder Stannylreste, ferner leicht abspaltbare 2-oxa- oder 2-thia-aliphatische oder -cycloaliphatische Kohlenwasserstoffreste, in erster Linie 1-Niederalkoxy-niederalkyl oder 1-Niederalkylthio-niederalkyl, z. B. 1-Methoxy-äthyl, 1-Äthoxy-äthyl, 1-Methylthio-äthyl oder 1-Äthylthioäthyl, oder 2-Oxa- oder 2-Thiacycloniederalkyl mit 5—7 Ringatomen, z. B. 2-Tetrahydrofuryl oder 2-Tetrahydropyranyl oder entsprechende Thiaanaloge, sowie leicht abspaltbare, gegebenenfalls substituierte $\alpha$-Phenylniederalkylreste, wie gegebenenfalls substituiertes Benzyl oder Diphenylmethyl, wobei als Substituenten der Phenylreste z. B. Halogen, wie Chlor, Niederalkoxy, wie Methoxy und/oder Nitro in Frage kommen.

Eine geschützte Carboxyl- oder Sulfogruppe ist in erster Linie eine mit einem aliphatischen, cycloaliphatischen, cycloaliphatisch-aliphatischen aromatischen oder araliphatischen Alkohol, wie einem Niederalkanol, oder mit einem Silyl- oder Stannylrest, wie Triniederalkylsilyl, veresterte Carboxyl- oder Sulfogruppe. In einer Carboxyl- oder Sulfogruppe kann die Hydroxygruppe beispielsweise wie die Hydroxygruppe in einer veresterten Carboxygruppe der Formel $-C(=O)-R_2$ veräthert sein.

O-substituiertes Hydroxyimino ist insbesondere Niederalkoxyimino, z. B. Methoxyimino oder Äthoxyimino, ferner Phenyloxyimino oder Phenylniederalkoxyimino, z. B. Benzyloxyimino, wobei solche Gruppen vorzugsweise in der syn-Form vorliegen.

Eine mit einer Aminoschutzgruppe $R_1^A$ geschützte Aminogruppe kann z. B. auch eine durch den Acylrest eines Kohlensäurehalbesters, eine 2-Carbonyl-vinyl-, Arylthio- oder Arylniederalkylthio- oder Arylsulfonylgruppe, einen Triarylmethylrest oder eine organische Silyl- oder Stannylgruppe geschützte Aminogruppe sein, wobei eine solche Schutzgruppe analog derjenigen einer entsprechend geschützten Aminogruppe in einem Acylrest der Formel IA sein kann.

Eine durch die Reste $R_1^a$ und $R_1^b$ zusammen gebildete bivalente Aminoschutzgruppe ist insbesondere der bivalente Acylrest einer organischen Dicarbonsäure, vorzugsweise mit bis zu 18 Kohlenstoffatomen, in erster Linie der Diacylrest einer aliphatischen oder aromatischen Dicarbonsäure, z. B. der Acylrest einer Niederalkan- oder Niederalkendicarbonsäure, wie Succinyl, oder einer o-Arylendicarbonsäure, wie Phthaloyl, oder ist ferner der Acylrest einer, in $\alpha$-Stellung vorzugsweise substituierten, z. B. einen aromatischen oder heterocyclischen Rest enthaltenden, $\alpha$-Aminoessigsäure, worin die Aminogruppe über einen, vorzugsweise substituierten, z. B. zwei Niederalkyl-, wie Methylgruppen enthaltenden Methylenrest mit dem Stickstoffatom verbunden ist, z. B. ein, insbesondere in 2-Stellung, substituierter, z. B. gegebenenfalls substituiertes Phenyl oder

Thienyl enthaltender, und in 4-Stellung gegebenenfalls durch Niederalkyl, wie Methyl, mono- oder disubstituierter 1-Oxo-3-aza-1,4-butylenrest, z. B. 4,4-Dimethyl-2-phenyl-1-oxo-3-aza-1,4-butylen.

Die Reste $R_1^a$ und $R_1^b$ können zusammen auch einen organischen, wie einen aliphatischen, cycloaliphatischen, cycloaliphatisch-aliphatischen oder araliphatischen Ylidenrest, vorzugsweise mit bis zu 18 Kohlenstoffatomen, darstellen.

Eine geschützte Carboxylgruppe der Formel $-C(=O)-R_2$ ist in erster Linie eine veresterte Carboxylgruppe, worin $R_2$ eine durch einen organischen Rest oder eine organische Silyl- oder Stannylgruppe verätherte Hydroxygruppe darstellt. Organische Reste, auch als Substituenten in organischen Silyl- oder Stannylgruppen, sind aliphatische, cycloaliphatische, cycloaliphatisch-aliphatische, aromatische oder araliphatische Reste, insbesondere gegebenenfalls substituierte Kohlenwasserstoffreste dieser Art, sowie heterocyclische oder heterocyclisch-aliphatische Reste, vorzugsweise mit bis zu 18 Kohlenstoffatomen.

Eine verätherte Hydroxygruppe $R_2$ bildet zusammen mit der Carbonylgruppierung eine, vorzugsweise leicht spaltbare, z. B. reduktiv, wie hydrogenolytisch, oder solvolytisch, wie acidolytisch oder hydrolytisch, sowie oxidativ spaltbare, oder leicht in eine andere funktionell abgewandelte Carboxylgruppe, wie in eine andere veresterte Carboxylgruppe oder in eine Hydrazinocarbonylgruppe umwandelbare, veresterte Carboxylgruppe. Eine solche Gruppe $R_2$ ist z. B. 2-Halogen-niederalkoxy, worin Halogen vorzugsweise ein Atomgewicht von über 19 hat, z. B. 2,2,2-Trichloräthoxy oder 2-Jodäthoxy, ferner 2-Chloräthoxy oder 2-Bromäthoxy, das sich leicht in letzteres überführen läßt, oder 2-Niederalkylsulfonylniederalkoxy, z. B. 2-Methylsulfonyläthoxy. Die Gruppe $R_2$ ist ferner eine, durch gegebenenfalls substituierte Kohlenwasserstoffreste, insbesondere gesättigte aliphatische oder aromatische Kohlenwasserstoffreste, wie Niederalkyl, z. B. Methyl und/oder Phenyl, polysubstituierte oder eine durch einen ungesättigten aliphatischen Kohlenwasserstoffrest, wie Niederalkenyl, z. B. 1-Niederalkenyl, wie Vinyl, durch eine, Elektronen-abgebende Substituenten aufweisende, carbocyclische Arylgruppe oder eine, Sauerstoff oder Schwefel als Ringglied aufweisende, heterocyclische Gruppe aromatischen Charakters, monosubstituierte Methoxygruppe, wie tert.-Niederalkoxy, z. B.-Butyloxy oder tert.-Pentyloxy, gegebenenfalls substituiertes Diphenylmethoxy, z. B. Diphenylmethoxy oder 4,4'-Dimethoxydiphenylmethoxy, Niederalkenyloxy, insbesondere 2-Niederalkenyloxy, z. B. Allyloxy, Niederalkoxy-phenylniederalkoxy, z. B. Niederalkoxy-benzyloxy, wie Methoxybenzyloxy (wobei Methoxy in erster Linie in 3-, 4- und/oder 5-Stellung steht), in erster Linie 3- oder 4-Methoxybenzyloxy, 3,4-Dimethoxybenzyloxy, oder vor allem Nitrobenzyloxy, z. B. 4-Nitrobenzyloxy, 2-Nitrobenzyloxy oder 4,5-Dimethoxy-2-nitro-benzyloxy, bzw. Furfuryloxy, wie 2-Furfuryloxy. $R_2$ kann auch 2-Oxa- oder 2-Thiacycloalkoxy oder -cycloalkenyloxy mit 5—7 Ringgliedern, wie 2-Tetrahydrofuryloxy, 2-Tetrahydropyranyloxy oder 2,3-Dihydro-2-pyranyloxy oder eine entsprechende Thiagruppe, oder Arylcarbonylmethoxy, worin Aryl insbesondere für eine gegebenenfalls substituierte Phenylgruppe steht, z. B. Phenacyloxy, sein oder bildet zusammen mit der $-C(=O)$-Gruppierung eine aktivierte Estergruppe und ist beispielsweise Nitrophenyloxy, z. B. 4-Nitrophenyloxy oder 2,4-Dinitrophenyloxy, oder Polyhalogenphenyloxy, z. B. Pentachlorphenyloxy. $R_2$ kann aber auch unverzweigtes Niederalkoxy, z. B. Methoxy oder Äthoxy sein.

Eine organische Silyloxy- oder organische Stannyloxygruppe $R_2$ ist insbesondere eine durch 1 bis 3 gegebenenfalls substituierte Kohlenwasserstoffreste, vorzugsweise mit bis zu 18 Kohlenstoffatomen, substituierte Silyloxy- oder Stannyloxygruppe. Sie enthält als Substituenten vorzugsweise gegebenenfalls substituierte, beispielsweise durch Niederalkoxy, wie Methoxy, oder durch Halogen, wie Chlor, substituierte aliphatische, cycloaliphatische, aromatische oder araliphatische Kohlenwasserstoffreste, wie Niederalkyl, Halogen-niederalkyl, Cycloalkyl, Phenyl oder Phenylniederalkyl, und stellt in erster Linie Triniederalkylsilyloxy, z. B. Trimethylsilyloxy, Halogen-niederalkoxyniederalkylsilyloxy, z. B. Chlor-methoxy-methyl-silyloxy, oder Triniederalkylstannyloxy, z. B. Tri-n-butylstannyloxy, dar.

Die Gruppe $R_2$ kann auch eine verätherte Hydroxygruppe sein, die zusammen mit der Carbonylgruppierung $-C(=O)-$ eine unter physiologischen Bedingungen spaltbare veresterte Carboxylgruppe bildet, in erster Linie eine Acyloxymethoxygruppe, worin Acyl z. B. den Rest einer organischen Carbonsäure, in erster Linie einer gegebenenfalls substituierten Niederalkancarbonsäure bedeutet, oder worin Acyloxymethyl den Rest eines Lactons bildet. So verätherte Hydroxygruppen sind Niederalkanoyloxy-methoxy, z. B. Acetyloxymethyloxy oder Pivaloyloxymethoxy, Amino-niederalkanoyloxymethoxy, insbesondere $\alpha$-Aminoniederalkanoyloxymethoxy, z. B. Glycyloxymethoxy, L-Valyloxymethoxy, L-Leucyloxymethoxy, ferner Phthalidyloxy.

Ein zusammen mit einer $-C(=O)$-Gruppierung eine gegebenenfalls substituierte Hydrazinocarbonylgruppe bildender Rest $R_2$ ist z. B. Hydrazino oder 2-Niederalkylhydrazino, z. B. 2-Methylhydrazino.

Salze sind insbesondere diejenigen von Verbindungen der Formel I oder II mit einer sauren Gruppierung, wie einer Carboxygruppe, in erster Linie Metall- oder Ammoniumsalze, wie Alkalimetall- und Erdalkalimetall-, z. B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische und araliphatische primäre, sekundäre oder tertiäre Mono-, Di- oder Polyamine, sowie heterocyclische Basen für die Salzbildung in Frage kommen, wie Niederalkylamine, z. B. Triäthylamin, Hydroxyniederalkylamine, z. B. 2-Hydroxyäthylamin, Di-(2-hydroxyäthyl)-amin oder Tri-(2-hydroxyäthyl)-amin, basische aliphatische Ester von Carbonsäuren, z. B. 4-Aminobenzoesäure-2-diäthylamino-äthylester, Niederalkylenamine, z. B. 1-Äthyl-piperidin, Cycloalkylamine, z. B. Bicyclohexylamin, oder Benzylamine, z. B. N,N'-Dibenzyläthylendiamin, ferner Basen vom Pyridintyp, z. B. Pyridin, Collidin oder Chinolin. Verbindungen der Formel I oder II, die eine basische Gruppe aufweisen, können ebenfalls Säureadditionssalze, z. B. mit anorganischen Säuren, wie Salzsäure, Schwefelsäure oder Phosphorsäure, oder mit geeigneten organischen Carbon- oder Sulfonsäuren, z. B. Trifluoressigsäure oder p-Toluolsulfonsäure, bilden. Verbindungen der Formel I oder II mit einer sauren und einer basischen Gruppe können auch in Form von inneren Salzen, d. h. in zwitterionischer Form, vorliegen. 1-Oxide von Verbindungen der Formel I oder II mit salzbildenden Gruppen können ebenfalls Salze, wie oben beschrieben, bilden. In einem Ausgangsmaterial der Formel II sind diejenigen Salze bevorzugt, die die Reduktion nicht stören.

Die Erfindung betrifft insbesondere die Herstellung von Verbindungen der Formel I, worin $R_1^a$ Wasserstoff oder vorzugsweise einen, in einem fermentativ (d. h. natürlich vorkommenden) oder bio-, halb- oder totalsynthetisch herstellbaren N-Acylderivat einer 6$\beta$-Amino-penam-3-carbonsäure- oder 7$\beta$-Amino-3-cephem-4-carbonsäureverbindung enthaltenen Acylrest, wie einen der obengenannten Acylreste der Formel (IA), bedeutet, wobei in dieser $R_a$, $R_b$ und $R_c$ in erster Linie die hervorgehobenen Bedeutungen haben, $R_1^b$ für Wasserstoff steht, oder worin $R_1^a$ und $R_1^b$ zusammen einen in 2-Stellung vorzugsweise, z. B. durch einen aromatischen oder heterocyclischen Rest, wie Phenyl, und in 4-Stellung vorzugsweise, z. B. durch zwei Niederalkyl, wie Methyl, substituierten 1-Oxo-3-aza-1,4-butylenrest darstellen, $R_2$ für eine durch einen organischen Rest oder eine organische Silyl- oder Stannylgruppe verätherte Hydroxygruppe oder für eine gegebenenfalls substituierte Hydrazinogruppe steht, von 1-Oxiden davon, sowie Salzen von solchen Verbindungen mit salzbildenden Gruppen, aus entsprechend substituierten Verbindungen der Formel II, deren 1-Oxiden oder Salzen.

In erster Linie steht in einer Verbindung der Formel I oder II, in einem 1-Oxid oder in einem Salz einer solchen Verbindung mit salzbildenden Gruppen $R_1^a$ für Wasserstoff oder einen Acylrest der Formel IA, worin (1) $R_a$ in erster Linie die hervorgehobenen Bedeutungen hat, und beispielsweise gegebenenfalls durch Hydroxy, geschütztes Hydroxy, Niederalkoxy, Niederalkanoyloxy, Carbamoyloxy, Halogen, Niederalkylsulfonylamino oder Aminomethyl substituiertes Phenyl, Thienyl, Furyl, Cyclohexadienyl oder Cyclohexenyl, oder durch Amino, Niederalkylamino oder geschütztes Amino substituiertes Thiazolyl, darstellt, $R_b$ Wasserstoff und $R_c$ Wasserstoff, gegebenenfalls geschütztes Hydroxy, gegebenenfalls geschütztes Amino oder gegebenenfalls geschütztes Carboxyl oder Sulfo darstellt, oder worin (2) $R_a$ gegebenenfalls geschütztes 3-Amino-3-carboxy-propyl, Cyan, 1-Tetrazolyl, gegebenenfalls wie Phenyl substituiertes Phenyloxy, oder 4-Pyridylthio und $R_b$ und $R_c$ Wasserstoff darstellen, oder worin (3) $R_a$ Phenyl, Thienyl, Furyl oder durch Amino, Niederalkylamino oder geschütztes Amino substituiertes Thiazolyl darstellt und $R_b$ und $R_c$ zusammen syn-Niederalkoxyimino bedeuten, $R_1^b$ für Wasserstoff steht und $R_2$ gegebenenfalls $\alpha$-poly-verzweigtes Niederalkoxy, z. B. Methoxy oder tert.-Butyloxy, oder 2-Halogen-niederalkoxy, z. B. 2,2,2-Trichloräthoxy, 2-Jodäthoxy oder das leicht in dieses überführbare 2-Chloräthoxy oder 2-Bromäthoxy, ferner Phenacyloxy, 1-Phenylniederalkoxy mit 1—3, gegebenenfalls durch Niederalkoxy und/oder Nitro substituierten Phenylresten, z. B. 4-Methoxybenzyloxy, 4-Nitrobenzyloxy, 2-Nitro-4,5-dimethoxybenzyloxy, Diphenylmethoxy, 4,4'-Dimethoxy-diphenylmethoxy oder Trityloxy, Niederalkanoyloxymethoxy, z. B. Acetyloxymethoxy oder Pivaloyloxymethoxy, $\alpha$-Aminoniederalkanoyloxymethoxy, z. B. Glycyloxymethoxy, 2-Phthalidyloxy, sowie Niederalkenyloxy, insbesondere 2-Niederalkenyloxy, z. B. Allyloxy, bedeutet.

Die Erfindung betrifft in erster Linie die Herstellung von Verbindungen der Formel I, worin $R_1^a$ Wasserstoff oder insbesondere eine Acylgruppe der Formel IA darstellt, worin (1) $R_a$ Phenyl, Hydroxyphenyl, z. B. 3- oder 4-Hydroxyphenyl, Niederalkylsulfonylaminophenyl, z. B. 3-Methylsulfonylamino-phenyl, Aminomethylphenyl, z. B. 2-Aminomethylphenyl, Thienyl, z. B. 2- oder 3-Thienyl, Aminomethylthienyl, z. B. 5-Aminomethyl-2-thienyl, Furyl, z. B. 2-Furyl, Aminomethylfuryl, z. B. 5-Aminomethyl-2-furyl, 1,4-Cyclohexadienyl, Aminomethyl-1,4-cyclohexadienyl, z. B. 2-Aminomethyl-1,4-cyclohexadienyl, 1-Cyclohexenyl, Aminomethyl-1-cyclohexenyl, z. B. 2-Aminomethyl-1-cyclohexenyl, 2-Amino-4-thiazolyl oder 2-Niederalkylamino-4-thiazolyl bedeutet, wobei in den obigen Resten Hydroxy und/oder Amino z. B. durch Acyl, wie gegebenenfalls halogeniertes Niederalkoxycarbonyl, z. B. tert.-Butyloxycarbonyl oder 2,2,2-Trichloräthoxycarbonyl, geschützt sein kann, $R_b$ für Wasserstoff und $R_c$ für Wasserstoff, für Amino, sowie geschütztes Amino, wie Acylamino, z. B. $\beta$-polyverzweigtes Niederalkoxycarbonylamino, wie tert.-Butyloxycarbonylamino oder 2-Halogenniederalkoxycarbonylamino, z. B. 2,2,2-Trichloräthoxycarbonylamino, 2-Jodäthoxycarbonylamino oder 2-Bromäthoxycarbonylamino, oder gegebenenfalls Niederalkoxy- und/oder nitrosubstituiertes Phenylniederalkoxycarbonylamino, z. B. 4-Methoxybenzyloxycarbonylamino oder Diphenylmethoxycarbonylamino, oder für Hydroxy, sowie geschütztes Hydroxy, wie Acyloxy, z. B. $\beta$-polyverzweigtes Niederalkoxycarbonyloxy, wie tert.-Butyloxycarbonyloxy, oder 2-Halogenniederalkoxycarbonyloxy, wie 2,2,2-Trichloräthoxycarbonyloxy, 2-Jodäthoxycarbonyloxy oder 2-Bromäthoxycarbonyloxy, oder für gegebenenfalls, z. B. mit Niederalkyl verestertes, Carboxyl oder Sulfo steht, oder worin (2) $R_a$ 3-Amino-3-carboxypropyl, worin Amino z. B. wie die obige Aminogruppe $R_c$ und Carboxy z. B. wie die 4-Carboxygruppe $-C(=O)-R_2$ geschützt sein können, Cyan, 1-Tetrazolyl, Phenyloxy oder 4-Pyridylthio und $R_b$ und $R_c$ Wasserstoff darstellen, oder worin (3) $R_a$ Phenyl, 2-Thienyl, 2-Furyl, 2-Amino-4-thiazolyl, 2-Niederalkylamino-, z. B.

2-Äthylamino-4-thiazolyl, worin Amino z. B. durch Acyl, wie gegebenenfalls halogeniertes Niederalkoxycarbonyl, z. B. tert.-Butyloxycarbonyl oder 2,2,2-Trichloräthoxycarbonyl, geschützt sein kann, darstellen und $R_b$ und $R_c$ zusammen syn-Niederalkoxyimino, wie syn-Methoxyimino, bedeuten, $R_1^b$ Wasserstoff bedeutet, und $R_2$ in erster Linie für Methoxy, $\alpha$-polyverzweigtes Niederalkoxy, z. B. tert.-Butyloxy, 2-Halogenniederalkoxy, z. B. 2,2,2-Trichloräthoxy, 2-Jodäthoxy oder 2-Bromäthoxy, oder gegebenenfalls, z. B. durch Niederalkoxy, wie Methoxy, substituiertes Diphenylmethoxy, z. B. Diphenylmethoxy oder 4,4'-Dimethoxydiphenylmethoxy, oder 4-Nitrobenzyloxy, ferner Triniederalkylsilyloxy, z. B. Trimethylsilyloxy, sowie 2-Niederalkenyloxy, z. B. Allyloxy, steht, sowie von deren 1-Oxiden und Salzen, aus entsprechend substituierten Verbindungen der Formel II, deren 1-Oxiden oder Salzen.

Die Erfindung betrifft in erster Linie die Herstellung von $7\beta$-Acetylamino-3-hydroxycepham-4-carbonsäure-Verbindungen, worin Acetyl durch die Reste $R_a$, $R_b$ und $R_c$ substituiert ist, wobei (1) $R_a$ für Phenyl, 2- oder 3-Thienyl, 2- oder 3-Furyl, $R_b$ für Wasserstoff oder gegebenenfalls, z. B. wie oben beschrieben, geschütztes Amino und $R_c$ für Wasserstoff stehen, oder worin (2) $R_a$ für Phenyloxy und $R_b$ und $R_c$ je für Wasserstoff stehen, oder worin (3) $R_a$ Phenyl oder 2-Amino-4-thiazolyl, 2-Niederalkylamino-, z. B. 2-Äthylamino-4-thiazolyl, worin Amino z. B. durch Acyl, wie gegebenenfalls halogeniertes Niederalkoxycarbonyl, z. B. tert.-Butyloxycarbonyl oder 2,2,2-Trichloräthoxycarbonyl, geschützt sein kann, darstellen, und $R_b$ und $R_c$ zusammen syn-Niederalkoxyimino, z. B. syn-Methoxyimino, bedeuten und $R_2$ eine veresterte Hydroxygruppe, insbesondere Niederalkoxy, z. B. Methoxy oder tert.-Butoxy, 2-Halogenniederalkoxy, z. B. 2,2,2-Trichloräthoxy, Nitrobenzyloxy, z. B. 4-Nitrobenzyloxy, oder Diphenylmethoxy bedeutet, von 1-Oxiden und Salzen solcher Verbindungen mit salzbildenden Gruppen, aus entsprechend substituierten Verbindungen der Formel II, deren 1-Oxiden oder Salzen.

Für das erfindungsgemäße Verfahren geeignete komplexe Borhydride sind beispielsweise Alkalimetallborhydride, z. B. Lithium- oder Natriumborhydrid, oder auch andere Metallborhydride, z. B. Zinkborhydrid, oder solche Borhydride, worin ein bis drei Wasserstoffatome durch Cyano oder durch Acyloxygruppen, beispielsweise durch gegebenenfalls halogenierte Alkanoyloxygruppen, z. B. Formyloxy, Acetyloxy, Propionyloxy, Palmityloxy, Monochloracetyloxy oder Trifluoracetyloxy, oder durch aromatische Acyloxygruppen, z. B. Benzoyloxy, oder dergleichen, oder auch durch Niederalkoxy, z. B. Methoxy oder Äthoxy, ersetzt sind, z. B. Natrium-cyano-borhydrid ($NaBH_3CN$), Natrium-triacetyloxyborhydrid [$NaBH(CH_3COO)_3$] oder Natrium-trimethoxyborhydrid [$NaBH(OCH_3)_3$]. Das Borhydrid wird, bezogen auf das Ausgangsmaterial der Formel II, in mindestens äquivalenter Menge, bevorzugt in einer Menge von 1 bis 10 Äquivalenten, gegebenenfalls portionenweise, eingesetzt.

Die erfindungsgemäß verwendbaren organischen Säuren sind unsubstituierte oder durch Halogen substituierte $C_1$—$C_7$-Niederalkancarbonsäuren, z. B. Ameisen-, Essig-, Propion-, Butter-, Chloressig- oder Trifluoressigsäure.

Bevorzugt sind die bei der Reaktionstemperatur flüssigen organischen Säuren, insbesondere die gegebenenfalls halogenierten Niederalkancarbonsäuren, wie die Ameisensäure, Trifluoressigsäure, und insbesondere die Essigsäure. Die genannten Säuren werden in Mengen von mindestens einem Mol pro Mol eingesetzter Verbindung der Formel II benutzt, bevorzugt in großem Überschuß, gegebenenfalls als Lösungsmittel.

Die erfindungsgemäße Reduktion wird entweder in einer der obengenannten organischen flüssigen Säuren allein oder unter Zusatz eines weiteren Lösungsmittels durchgeführt. Als zusätzliche Lösungsmittel geeignet sind inerte, die Reduktion nicht störende, insbesonders polare, Lösungsmittel, wie halogenierte Kohlenwasserstoffe, z. B. Methylenchlorid, Äther, z. B. Diäthyläther, Niederalkylenglycoldiniederalkyläther, z. B. Dimethoxyäthan oder Diäthylenglycoldimethyläther, cyclische Äther, z. B. Dioxan oder Tetrahydrofuran, Carbonsäureamide, z. B. Dimethylformamid, Diniederalkylsulfoxide, z. B. Dimethylsulfoxid, oder Niederalkanole, z. B. Methanol, Äthanol oder tert.-Butanol, oder Gemische davon. Falls notwendig, wird in einer Intertgas-, z. B. Argon- oder Stickstoffatmosphäre, gearbeitet.

Die Reaktionstemperatur liegt zwischen $-20°$ und $+80°$ und bevorzugt zwischen 0° und 30°.

Eine bevorzugte Variante stellt die Reduktion mit Natriumborhydrid in Gegenwart von Ameisensäure oder Eisessig bei etwa $+15°$ bis $+20°$ dar.

In erfindungsgemäß erhältlichen Verbindungen kann man funktionelle Substituenten in Resten $R_1^A$, $R_1^b$ und/oder $R_2$ in andere funktionelle Gruppen umwandeln.

Erhaltene Verbindungen der Formel I können in an sich bekannter Weise durch Oxydation mit geeigneten Oxydationsmitteln, wie Wasserstoffperoxid oder Persäuren, z. B. Peressigsäure oder 3-Chlorperbenzoesäure, in ihre 1-Oxide übergeführt werden. Erhaltene 1-Oxide von Verbindungen der Formel I lassen sich in an sich bekannter Weise durch Reduktion mit geeigneten Reduktionsmitteln, wie Phosphortrichlorid, zu den entsprechenden Verbindungen der Formel I reduzieren. Bei diesen Reaktionen muß darauf geachtet werden, daß, wenn notwendig, freie funktionelle Gruppen geschützt sind und, wenn erwünscht, nachträglich wieder freigesetzt werden.

Salze von Verbindungen der Formel I können in an sicht bekannter Weise hergestellt werden. So kann man Salze von solchen Verbindungen mit sauren Gruppen z. B. durch Behandeln mit

Metallverbindungen, wie Alkalimetallsalzen von geeigneten Carbonsäuren, z. B. dem Natriumsalz der $\alpha$-Äthylcapronsäure, oder mit Ammoniak oder einem geeigneten organischen Amin bilden, wobei man vorzugsweise stöchiometrische Mengen oder nur einen kleinen Überschuß des salzbildenden Mittels verwendet. Säureadditionssalze von Verbindungen der Formel I mit basischen Gruppierungen erhält man in üblicher Weise, z. B. durch Behandeln mit einer Säure oder einem geeigneten Anionenaustauschreagens. Innere Salze von Verbindungen der Formel I, welche z. B. eine salzbildende Aminogruppe und eine freie Carboxylgruppe enthalten, können z. B. durch Neutralisieren von Salzen, wie Säureadditionssalzen, auf den isoelektrischen Punkt, z. B. mit schwachen Basen, oder durch Behandeln mit flüssigen Ionenaustauschern gebildet werden. Salze von 1-Oxyden von Verbindungen der Formel I mit salzbildenden Gruppen können in analoger Weise hergestellt werden.

Salze können in üblicher Weise in die freien Verbindungen übergeführt werden, Metall- und Ammoniumsalze z. B. durch Behandeln mit geeigneten Säuren, und Säureadditionssalze z. B. durch Behandeln mit einem geeigneten basischen Mittel.

Erhaltene Gemische von Isomeren können nach an sich bekannten Methoden, in die einzelnen Isomeren getrennt werden, Gemische von diastereomeren Isomeren z. B. durch fraktioniertes Kristallisieren, Adsorptionschromatographie (Kolonnen- oder Dünnschichtchromatographie) oder andere geeignete Trennverfahren. Erhaltene Racemate können in üblicher Weise, gegebenenfalls nach Einführen von geeigneten salzbildenden Gruppierungen, z. B. durch Bilden eines Gemisches von diastereoisomeren Salzen mit optisch aktiven salzbildenden Mitteln, Trennen des Gemisches in die diastereoisomeren Salze und Verwandlung der Salze in die freien Verbindungen oder durch fraktioniertes Kristallisieren aus optisch aktiven Lösungsmitteln, in die Antipoden getrennt werden.

Vorzugsweise werden solche Ausgangsstoffe verwendet und die Reaktionsbedingungen so gewählt, daß man zu den eingangs als besonders bevorzugt aufgeführten Verbindungen gelangt.

Die Ausgangsverbindungen der Formel II, 1-Oxide und Salze davon sind bekannt oder können auf an sich bekannter Weise, z. B. analog den Deutschen Offenlegungsschriften 2 331 148, 2 506 330 oder 2 606 196 hergestellt werden.

Die Endprodukte der Formel I, 1-Oxide und Salze davon können, beispielsweise analog der Österreichischen Patentschrift 327 381, in antibiotisch wirksame 7$\beta$-Acylamido-3-cephem-4-carbonsäuren übergeführt werden.

Die folgenden Beispiele dienen zur Illustration der Erfindung. Temperaturen werden in Celsiusgraden angegeben.

## Beispiel 1

### 3-Hydroxy-7$\beta$-phenoxyacetylamino-cepham-4$\alpha$-carbonsäure-diphenylmethylester

a) Eine Lösung von 35,4 g 3-Hydroxy-7$\beta$-phenoxyacetylamino-3-cephem-4-carbonsäure-diphenylmethylester (Rohprodukt) in einer Mischung von 270 ml Dimethylformamid und 70 ml Eisessig wird in einem Stickstoffstrom unter Rühren und Eiskühlung bei etwa +15° bis +20° portionenweise mit 1,30 g Natriumborhydrid versetzt. Die Reaktionsmischung wird während einer Stunde bei Raumtemperatur weitergerührt, auf eine Mischung von Eis und 2N Salzsäure gegossen und mit Äthylacetat extrahiert. Die organische Phase wird nacheinander mit 2N Salzsäure, Wasser, gesättigter wäßriger Natriumhydrogencarbonatlösung und gesättigter wäßriger Natriumchloridlösung gewaschen, über Natriumsulfat getrocknet und im Vakuum eingeengt. Der Rückstand wird am Hochvakuum weitgehend vom Dimethylformamid befreit und aus Äthylacetat und wenig Diäthyläther umkristallisiert. Man erhält die Titelverbindung vom Schmelzpunkt 167—170°; Rf-Wert ~0,47 (Silicagel; Toluol-Äthylacetat 1 : 1); $[\alpha]_D^{20} = +88° \pm 1°$ (in Chloroform, c=0,526%); IR-Spektrum (in $CH_2Cl_2$): Absorptionsbanden bis 3580; 3410; 1780; 1740; 1695; 1600; 1518; 1494 $cm^{-1}$.

### Alternative Reaktionsbedingungen

b) Eine Lösung von 5,16 g 3-Hydroxy-7$\beta$-phenoxyacetylamino-3-cephem-4-carbonsäure-diphenylmethylester (Rohprodukt) in 51 ml Eisessig wird in einem Stickstoffstrom unter Rühren und Eiskühlung bei etwa +15° portionenweise mit 940 mg (10 Äquivalente) Natriumborhydrid versetzt. Die Reaktionsmischung wird während einer Stunde bei Raumtemperatur weitergerührt, auf eine Mischung von Eis und 2N Salzsäure gegossen und mit Äthylacetat extrahiert. Die organische Phase wird mit Wasser und gesättigter wäßriger Natriumchloridlösung gewaschen, über Natriumsulfat getrocknet und im Vakuum eingeengt. Der Rückstand wird aus Methylenchlorid und Diäthyläther umkristallisiert. Man erhält die Titelverbindung mit den unter a) angegebenen Eigenschaften.

c) Auf die gleiche Weise wie unter b) angegeben, werden 516 mg 3-Hydroxy-7$\beta$-phenoxyacetylamino-3-cephem-4-carbonsäure-diphenylmethylester (Rohprodukt) in 5,1 ml Eisessig mit 94 mg Natriumborhydrid reduziert und aufgearbeitet. Man erhält die Titelverbindung mit den unter a) angegebenen Eigenschaften.

d) Auf die gleiche Weise wie unter a) angegeben, werden 111 g 3-Hydroxy-7β-phenoxyacetylamino-3-cephem-4-carbonsäure-diphenylmethylester (Rohprodukt) in einer Mischung von 640 ml Dimethylformamid und 160 ml Eisessig mit 4,5 g Natriumborhydrid reduziert und aufgearbeitet. Man erhält die Titelverbindung mit den unter a) angegebenen Eigenschaften.

e) Eine Lösung von 516 mg 3-Hydroxy-7β-phenoxyacetylamino-3-cephem-4-carbonsäure-diphenylmethylester in 4 ml Dimethylformamid und 1 ml Ameisensäure wird bei Raumtemperatur mit 28,4 mg Natriumborhydrid versetzt. Nach einstündigem Weiterrühren bei Raumtemperatur wird die Reaktionsmischung auf eine Mischung von Eis und 2N Salzsäure gegossen und mit Äthylacetat extrahiert. Die vereinigten organischen Phasen werden nacheinander mit Wasser, dreimal mit gesättigter Natriumhydrogencarbonatlösung und gesättigter, wäßriger Natriumchloridlösung gewaschen, über Natriumsulfat getrocknet und im Vakuum eingeengt. Der Rückstand wird am Hochvakuum weitgehend vom Dimethylformamid befreit. Der erhaltene weiße Schaum (580 mg) wird an präparativen Kieselgeldickschichtplatten mit Toluol-Äthylacetat 1 : 1 chromatographiert und ergibt 403 mg der Titelverbindung.

f) Ein analog und parallel zu e) durchgeführter Reduktionsversuch ohne die Ameisensäure, ergab nur 25 mg der Titelverbindung.

Herstellung des Ausgangsmaterials

Die in den Beispielen 1a) bis 1e) verwendeten Ausgangsmaterialien können beispielsweise wie folgt hergestellt werden:

i) Eine auf −10° gekühlte Lösung von 107,68 g 2-[4-(p-Toluolsulfonylthio)-3-phenoxyacetamido-2-oxoazetidin-1-yl]-3-hydroxycrotonsäure-diphenylmethylester in 1,6 Liter trockenem Methylenchlorid wird unter Stickstoff mit 24,8 ml Methansulfonylchlorid und anschließend mit 43,8 ml Triäthylamin versetzt. Nach 20 Minuten wird 40,8 ml frisch destilliertes Pyrrolidin zugefügt und weitere 2 1/2 Stunden bei −10° gerührt. Die Reaktionslösung wird nacheinander mit 0,1N Salzsäure, Wasser und gesättigter wäßriger Natriumchloridlösung gewaschen, über Natriumsulfat getrocknet und im Vakuum eingedampft. Der Rückstand wird zu einem Schaum getrocknet und ergibt eine Mischung bestehend aus dem leicht gelben 2-[4-(p-Toluolsulfonylthio)-3-phenoxyacetamido-2-oxoazetidin-1-yl]-3-(1-pyrrolidinyl)-crotonsäure-diphenylmethylester und dem entsprechenden Isocrotonsäure-diphenylmethylester, die in dieser Form in der nächsten Stufe eingesetzt wird.

ii) Eine Lösung von 139,32 g (160 m Mol) eines Gemisches, bestehend aus dem 2-[4-(p-Toluolsulfonylthio)-3-phenoxyacetamido-2-oxoazetidin-1-yl]-3-(1-pyrrolidinyl)-crotonsäure-diphenylmethylester und dem entsprechenden Isocrotonsäureester in 1350 ml trockenem Acetonitril wird unter Stickstoff etwa 6 Stunden bei 80° erhitzt, bis dünnschichtchromatographisch (Silikagel; Toluol/Äthylacetat 1 : 1) kein Ausgangsmaterial mehr nachweisbar ist. Das Heizbad wird entfernt, die Reaktionsmischung, enthaltend den 7β-Phenoxyacetamido-3-pyrrolidino-3-cephem-4-carbonsäure-diphenylmethylester wird noch 1 1/2 Stunden abkühlen gelassen, mit 550 ml 0,1 N Salzsäure versetzt und 8 1/2 Stunden bei Raumtemperatur weitergerührt. Die Reaktionsmischung wird mit Äthylacetat verdünnt, das Wasser wird abgetrennt, die organische Phase zweimal mit 2N Salzsäure und dreimal mit Wasser gewaschen, über Natriumsulfat getrocknet, im Vakuum eingedampft und am Hochvakuum getrocknet. Der Rückstand, enthaltend den 7β-Phenoxyacetamido-3-hydroxy-3-cephem-4-carbonsäure-diphenylmethylester wird ohne weitere Reinigung in die nächste Stufe eingesetzt.

Beispiel 2

Analog den in den Beispielen 1a) bis 1e) beschriebenen Verfahren können ausgehend von den entsprechenden 3-Hydroxy-3-cephem-4-carbonsäure-estern die folgenden Verbindungen erhalten werden:

7β-Phenyloxyacetylamino-3-hydroxycepham-4-carbonsäure-tert.butylester,
7β-Phenyloxyacetylamino-3-hydroxycepham-4-carbonsäure-trichloräthylester,
7β-Phenyloxyacetylamino-3-hydroxycepham-4-carbonsäure-benzylester,
7β-Phenyloxyacetylamino-3-hydroxycepham-4-carbonsäure-p-methoxybenzylester,
7β-Phenyloxyacetylamino-3-hydroxycepham-4-carbonsäure-p-nitrobenzylester,
7β-Phenylacetylamino-3-hydroxycepham-4-carbonsäure-diphenylmethylester,
7β-Phenylacetylamino-3-hydroxycepham-4-carbonsäure-tert.butylester,
7β-Phenylacetylamino-3-hydroxycepham-4-carbonsäure-trichloräthylester,
7β-Phenylacetylamino-3-hydroxycepham-4-carbonsäure-benzylester,
7β-Phenylacetylamino-3-hydroxycepham-4-carbonsäure-p-methoxybenzylester,

7β-Phenylacetylamino-3-hydroxycepham-4-carbonsäure-p-nitrobenzylester,
7β-Thien-2- oder -3-ylacetylamino-3-hydroxycepham-4-carbonsäure-diphenylmethylester,
7β-Thien-2- oder -3-ylacetylamino-3-hydroxycepham-4-carbonsäure-tert.butylester,
7β-Thien-2- oder -3-ylacetylamino-3-hydroxycepham-4-carbonsäure-trichloräthylester,
7β-Thien-2- oder -3-ylacetylamino-3-hydroxycepham-4-carbonsäure-benzylester,
7β-Thien-2- oder -3-ylacetylamino-3-hydroxycepham-4-carbonsäure-p-methoxybenzylester,
7β-Thien-2- oder -3-ylacetylamino-3-hydroxycepham-4-carbonsäure-p-nitrobenzylester,
7β-Fur-2- oder -3-ylacetylamino-3-hydroxycepham-4-carbonsäure-diphenylmethylester,
7β-Fur-2- oder -3-ylacetylamino-3-hydroxycepham-4-carbonsäure-tert.butylester,
7β-Fur-2- oder -3-ylacetylamino-3-hydroxycepham-4-carbonsäure-trichloräthylester,
7β-Fur-2- oder -3-ylacetylamino-3-hydroxycepham-4-carbonsäure-benzylester,
7β-Fur-2- oder -3-ylacetylamino-3-hydroxycepham-4-carbonsäure-p-methoxybenzylester,
7β-Fur-2- oder -3-ylacetylamino-3-hydroxycepham-4-carbonsäure-p-nitrobenzylester,
7β-(5-Diphenylmethoxycarbonyl-5-tert.butoxycarbonylamino-valerylamino)-3-hydroxycepham-4-carbonsäure-diphenylmethylester,
7β-(5-Diphenylmethoxycarbonyl-5-tert.butoxycarbonylamino-valerylamino)-3-hydroxycepham-4-carbonsäure-tert.butylester,
7β-(5-Diphenylmethoxycarbonyl-5-tert.butoxycarbonylamino-valerylamino)-3-hydroxycepham-4-carbonsäure-trichloräthylester,
7β-(5-Diphenylmethoxycarbonyl-5-tert.butoxycarbonylamino-valerylamino)-3-hydroxycepham-4-carbonsäure-benzylester,
7β-(5-Diphenylmethoxycarbonyl-5-tert.butoxycarbonylamino-valerylamino)-3-hydroxycepham-4-carbonsäure-p-methoxybenzylester,
7β-(5-Diphenylmethoxycarbonyl-5-tert.butoxycarbonylamino-valerylamino)-3-hydroxycepham-4-carbonsäure-p-nitrobenzylester,
7β-(D-N-tert.Butoxycarbonyl-phenylglycylamino)-3-hydroxycepham-4-carbonsäure-diphenylmethylester,
7β-(D-N-tert.Butoxycarbonyl-phenylglycylamino)-3-hydroxycepham-4-carbonsäure-tert.butylester,
7β-(D-N-tert.Butoxycarbonyl-phenylglycylamino)-3-hydroxycepham-4-carbonsäure-trichloräthylester,
7β-(D-N-tert.Butoxycarbonyl-phenylglycylamino)-3-hydroxycepham-4-carbonsäure-benzylester,
7β-(D-N-tert.Butoxycarbonyl-phenylglycylamino)-3-hydroxycepham-4-carbonsäure-p-methoxybenzylester,
7β-(D-N-tert.Butoxycarbonyl-phenylglycylamino)-3-hydroxycepham-4-carbonsäure-p-nitrobenzylester,
7β-[D-α-tert.Butoxycarbonylamino-α-(1,4-cyclohexadienyl)-glycylamino]-3-hydroxycepham-4-carbonsäure-diphenylmethylester,
7β-[D-α-tert.Butoxycarbonylamino-α-(1,4-cyclohexadienyl)-glycylamino]-3-hydroxycepham-4-carbonsäure-tert.butylester,
7β-[D-α-tert.Butoxycarbonylamino-α-(1,4-cyclohexadienyl)-glycylamino]-3-hydroxycepham-4-carbonsäure-trichloräthylester,
7β-[D-α-tert.Butoxycarbonylamino-α-(1,4-cyclohexadienyl)-glycylamino]-3-hydroxycepham-4-carbonsäure-benzylester,
7β-[D-α-tert.Butoxycarbonylamino-α-(1,4-cyclohexadienyl)-glycylamino]-3-hydroxycepham-4-carbonsäure-p-methoxybenzylester,
7β-[D-α-tert.Butoxycarbonylamino-α-(1,4-cyclohexadienyl)-glycylamino]-3-hydroxycepham-4-carbonsäure-p-nitrobenzylester,
7β-[2-(2-tert.Butoxycarbonylamino-4-thiazolyl)-2-methoxyiminoacetylamino]-3-hydroxycepham-4-carbonsäure-diphenylmethylester,
7β-[2-(2-tert.Butoxycarbonylamino-4-thiazolyl)-2-methoxyiminoacetylamino]-3-hydroxycepham-4-carbonsäure-tert.butylester,
7β-[2-(2-tert.Butoxycarbonylamino-4-thiazolyl)-2-methoxyiminoacetylamino]-3-hydroxycepham-4-carbonsäure-trichloräthylester,
7β-[2-(2-tert.Butoxycarbonylamino-4-thiazolyl)-2-methoxyiminoacetylamino]-3-hydroxycepham-4-carbonsäure-benzylester,
7β-[2-(2-tert.Butoxycarbonylamino-4-thiazolyl)-2-methoxyiminoacetylamino]-3-hydroxycepham-4-carbonsäure-p-methoxybenzylester,
7β-[2-(2-tert.Butoxycarbonylamino-4-thiazolyl)-2-methoxyiminoacetylamino]-3-hydroxycepham-4-carbonsäure-p-nitrobenzylester,
7β-Amino-3-hydroxycepham-4-carbonsäure-diphenylmethylester,
7β-Amino-3-hydroxycepham-4-carbonsäure-tert.butylester,
7β-Amino-3-hydroxycepham-4-carbonsäure-trichloräthylester,
7β-Amino-3-hydroxycepham-4-carbonsäure-benzylester,
7β-Amino-3-hydroxycepham-4-carbonsäure-p-methoxybenzylester,

7β-Amino-3-hydroxycepham-4-carbonsäure-p-nitrobenzylester, sowie, ausgehend von den entsprechenden 1-Oxiden, die 1-Oxide davon.

**Patentansprüche**

1. Verfahren zur Herstellung von 7β-substituierten-3-Hydroxycepham-4-carbonsäureverbindungen der Formel

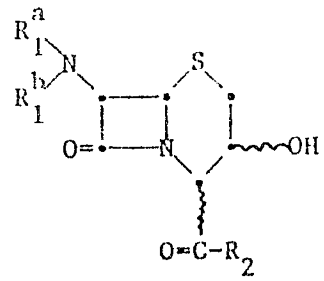

(I)

worin $R_1^a$ Wasserstoff oder eine Aminoschutzgruppe $R_1^A$ bedeutet und $R_1^b$ Wasserstoff oder eine Acylgruppe Ac darstellt, oder worin $R_1^a$ und $R_1^b$ zusammen eine bivalente Aminoschutzgruppe bilden und $R_2$ einen zusammen mit der Carbonylgruppierung $-C(=O)-$ eine geschützte Carboxylgruppe bildenden Rest bedeutet, von 1-Oxiden davon, sowie Salzen von solchen Verbindungen mit salzbildenden Gruppen, aus einer 7β-substituierten-3-Hydroxy-3-cephem-4-carbonsäureverbindung der Formel

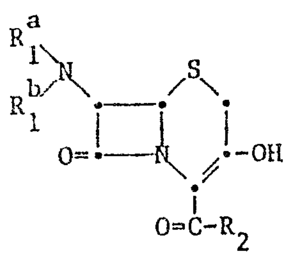

(II)

aus einem 1-Oxid oder einem Salz davon, und einem komplexen Borhydrid, dadurch gekennzeichnet, daß man die Reduktion in Gegenwart einer unsubstituierten oder durch Halogen substituierten $C_1-C_7$-Niederalkancarbonsäure durchführt.

2. Verfahren zur Herstellung von Verbindungen der Formel I, worin $R_1^a$ Wasserstoff oder eine Acylgruppe Ac und $R_1^b$ Wasserstoff bedeutet, nach Patentanspruch 1.

3. Verfahren zur Herstellung von Verbindungen der Formel I, worin $R_2$ einen zusammen mit der Carbonylgruppierung $-C(=O)-$ eine veresterte Carboxylgruppe bildenden Rest bedeutet, nach Patentanspruch 1.

4. Verfahren nach Patentanspruch 1 zur Herstellung von Verbindungen der Formel I, worin $R_1^a$ Wasserstoff oder eine Acylgruppe der Formel

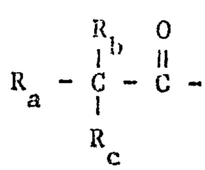

(IA)

darstellt, worin (I) $R_a$ Phenyl, Hydroxyphenyl, Niederalkylsulfonylaminophenyl, Aminomethylphenyl, Thienyl, Aminomethylthienyl, Furyl, Aminomethylfuryl, 1,4-Cyclohexadienyl, Aminomethyl-1,4-cyclohexadienyl, 1-Cyclohexenyl, Aminomethyl-1-cyclohexenyl, 2-Amino-4-thiazolyl oder 2-Niederalkylamino-4-thiazolyl bedeutet, wobei Hydroxy und/oder Amino in solchen Resten geschützt sein kann, $R_b$ für Wasserstoff und $R_c$ für Wasserstoff, Amino, geschütztes Amino, Hydroxy, geschütztes Hydroxy, oder für gegebenenfalls verestertes Carboxyl oder Sulfo steht, oder worin (2) $R_a$ 3-Amino-3-carboxypropyl, worin Amino und Carboxy geschützt sein können, Cyan, 1-Tetrazolyl, Phenyloxy oder 4-Pyridylthio und $R_b$ und $R_c$ Wasserstoff darstellen, oder worin (3) $R_a$ Phenyl, Thienyl, Furyl, 2-Amino-4-thiazolyl oder 2-Niederalkylamino-4-thiazolyl, worin Amino geschützt sein kann, darstellen und $R_b$ und $R_c$ zusammen syn-Niederalkoxyimino bedeuten, $R_1^b$ Wasserstoff bedeutet, und $R_2$ für Methoxy, α-polyverzweigtes

Niederalkoxy, 2-Halogenniederalkoxy, gegebenenfalls durch Niederalkoxy substituiertes Diphenylmethoxy, 4-Nitrobenzyloxy, Triniederalkylsilyloxy oder 2-Niederalkenyloxy steht, und worin die mit »nieder« bezeichneten Gruppen 1—7 Kohlenstoffatome aufweisen.

5. Verfahren nach einem der Patentansprüche 1 bis 4 unter Verwendung von einem Alkalimetallborhydrid.

6. Verfahren nach einem der Patentansprüche 1 bis 5 unter Verwendung von Natriumborhydrid.

7. Verfahren nach einem der Patentansprüche 1 bis 6, dadurch gekennzeichnet, daß man die Reduktion mit Natriumborhydrid in Gegenwart von Eisessig durchführt.

8. Verfahren nach einem der Patentansprüche 1 bis 7, dadurch gekennzeichnet, daß man die Reduktion mit Natriumborhydrid in Gegenwart von Ameisensäure durchführt.

9. Verfahren nach einem der Patentansprüche 1 bis 8, dadurch gekennzeichnet, daß man die Reduktion bei ca. +15° bis ca. +20° C durchführt.

10. Verfahren nach einem der Patentansprüche 1 bis 9, zur Herstellung von 7β-Phenoxyacetylamino-3-hydroxycepham-4α-carbonsäure-diphenylmethylester mit Natriumborhydrid, dadurch gekennzeichnet, daß man die Reaktion in Gegenwart von Eisessig durchführt.

11. Verfahren nach einem der Patentansprüche 1 bis 9, zur Herstellung von 7β-Phenoxyacetylamino-3-hydroxycepham-4α-carbonsäure-diphenylmethylester mit Natriumborhydrid, dadurch gekennzeichnet, daß man die Reaktion in Gegenwart von Ameisensäure durchführt.

## Claims

1. A process for the production of 7β-substituted 3-hydroxycepham-4-carboxylic acid compounds of the formula

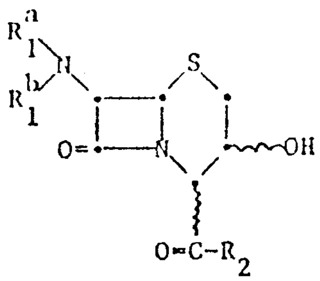

(I)

wherein $R_1^a$ is hydrogen or an amino-protecting group $R_1^A$, and $R_1^b$ is hydrogen or an acyl group Ac, or wherein $R_1^a$ and $R_1^b$ together form a bivalent amino-protecting group, and $R_2$ is a radical that, together with the carbonyl grouping $-C(=O)-$, forms a protected carboxyl group; and 1-oxides thereof, as well as salts of such compounds with salt-forming groups, from a 7β-substituted 3-hydroxy-3-cephem-4-carboxylic acid compound of the formula

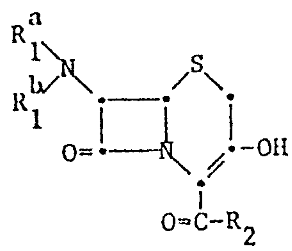

(II)

from a 1-oxide or a salt thereof, and a complex borohydride, characterised in that the reduction is carried out in the presence of a $C_1-C_7$-lower alkanecarboxylic acid which is unsubstituted or substituted by halogen.

2. A process for the production of compounds of the formula I, wherein $R_1^a$ is hydrogen or an acyl group Ac, and $R_1^b$ is hydrogen, according to claim 1.

3. A process for the production of compounds of the formula I, wherein $R_2$ is a radical that, together with the carbonyl grouping $-C(=O)-$, forms an esterified carboxyl group, according to claim 1.

4. A process according to claim 1 for the production of compounds of the formula I, wherein $R_1^a$ is hydrogen or an acyl group of the formula

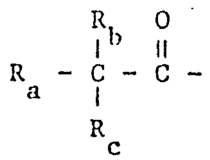

(IA)

wherein (I) $R_a$ is phenyl, hydroxyphenyl, lower alkylsulfonylaminophenyl, aminomethylphenyl, thienyl, aminomethylthienyl, furyl, aminomethylfuryl, 1,4-cyclohexadienyl, aminomethyl-1,4-cyclohexadienyl, 1-cyclohexenyl, aminomethyl-1-cyclohexenyl, 2-amino-4-thiazolyl or 2-lower alkylamino-4-thiazolyl, whereby hydroxyl and/or amino can be protected in such radicals, $R_b$ is hydrogen, and $R_c$ is hydrogen, amino, protected amino, hydroxyl, protected hydroxyl, or free or esterified carboxyl or sulfo, or wherein (2) $R_a$ is 3-amino-3-carboxypropyl, in which amino and carboxyl can be protected, cyano, 1-tetrazolyl, phenyloxy or 4-pyridylthio, and $R_b$ and $R_c$ are hydrogen, or wherein (3) $R_a$ is phenyl, thienyl, furyl, 2-amino-4-thiazolyl or 2-lower alkylamino-4-thiazolyl, in which amino can be protected, and $R_b$ and $R_c$ together are syn-lower alkoxyimino, $R_1^b$ is hydrogen, and $R_2$ is methoxy, $\alpha$-poly-branched lower alkoxy, 2-halo-lower alkoxy, diphenylmethoxy unsubstituted or substituted by lower alkoxy, or it is 4-nitrobenzyloxy, tri-lower-alkylsilyloxy or 2-loweralkenyloxy, and wherein the groups denoted by the term »lower« have 1—7 carbon atoms.

5. A process according to any one of claims 1 to 4 with the use of an alkali metal borohydride.

6. A process according to any one of claims 1 to 5 with the use of sodium borohydride.

7. A process according to any one of claims 1 to 6, characterised in that the reduction is carried out with sodium borohydride in the presence of glacial acetic acid.

8. A process according to any one of claims 1 to 7, characterised in that the reduction is carried out with sodium borohydride in the presence of formic acid.

9. A process according to any one of claims 1 to 8, wherein the reduction is carried out at about +15° to about +20° C.

10. A process according to any one of claims 1 to 9 for the production of 7$\beta$-phenoxyacetylamino-3-hydroxycephem-4$\alpha$-carboxylic acid diphenylmethyl ester with sodium borohydride, characterised in that the reaction is performed in the presence of glacial acetic acid.

11. A process according to any one of claims 1 to 9 for the production of 7$\beta$-phenoxyacetylamino-3-hydroxycephem-4$\alpha$-carboxylic acid diphenylmethyl ester with sodium borohydride, characterised in that the reaction is performed in the presence of formic acid.

## Revendications

1. Procédé de préparation de dérivés d'acides 3-hydroxycéphame-4-carboxyliques substitués en 7$\beta$, de formule

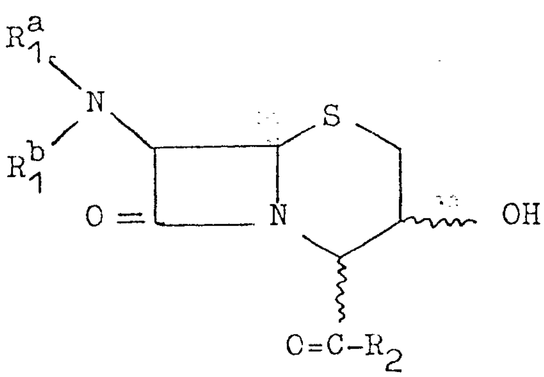

(I)

dans laquelle $R_1^a$ représente l'hydrogène ou un groupe protecteur d'un groupe amino, $R_1^A$ et $R_1^b$ représentent l'hydrogène ou un groupe acyle Ac, ou bien $R_1^a$ et $R_1^b$ forment ensemble un groupe bivalent protecteur d'un groupe amino et $R_2$ forme avec le groupement carbonyle $-C(=O)-$ un reste formant un groupe carboxyle protégé, de leurs 1-oxydes et des sels de ces composés portant des groupes salifiables, à partir d'un dérivé d'acide 3-hydroxy-3-céphème-4-carboxylique substitué en 7$\beta$, de formule

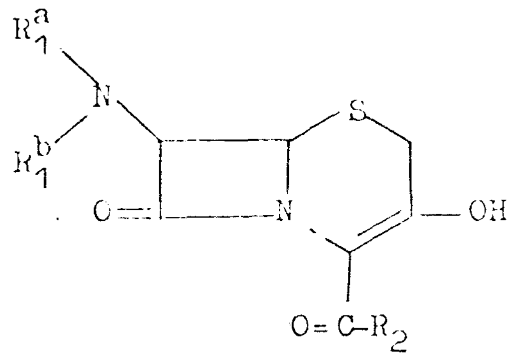

(II)

à partir d'un 1-oxyde ou d'un sel d'un tel composé, et d'un borohydrure complexe, ce procédé se caractérisant en ce que la réduction est effectuée en présence d'un acide (alcane inférieur en $C_1-C_7$)-carboxylique non substitué ou substitué par des halogènes.

2. Procédé de préparation des composés de formule I dans laquelle $R_1^a$ représente l'hydrogène ou un groupe acyle Ac et $R_1^b$ représente l'hydrogène, selon la revendication 1.

3. Procédé de préparation des composés de formule I dans laquelle $R_2$ forme avec le groupement carbonyle $-C(=O)-$ un reste formant un groupe carboxyle estérifié, selon la revendication 1.

4. Procédé selon la revendication 1, pour préparer les composés de formule I dans laquelle $R_1^a$ représente l'hydrogène ou un groupe acyle de formule

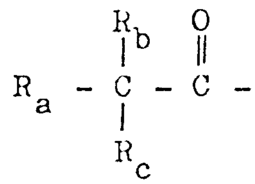

(IA)

dans laquelle (1) $R_a$ représente un groupe phényle, hydroxyphényle, (alkyle inférieur)-sulfonylamino-phényle, aminométhylphényle, thiényle, aminométhylthiényle, furyle, aminométhylfuryle, 1,4-cyclohexadiényle, aminométhyl-1,4-cyclohexadiényle, 1-cyclohexényle, aminométhyl-1-cyclohexényle, 2-amino-4-thiazolyle, ou 2-(alkyle inférieur)-amino-4-thiazolyle, les groupes hydroxy et/ou amino de ces restes pouvant être protégés, $R_b$ représente l'hydrogène et $R_c$ représente l'hydrogène, un groupe amino, amino protégé, hydroxy, hydroxy protégé, ou un groupe carboxyle ou sulfo éventuellement estérifié, ou bien (2) $R_a$ représente un groupe 3-amino-3-carboxypropyle dans lequel le groupe amino et le groupe carboxy peuvent être protégés, cyano, 1-tétrazolyle, phényloxy ou 4-pyridylthio et $R_b$ et $R_c$ représentent l'hydrogène, ou bien (3) $R_a$ représente un groupe phényle, thiényle, furyle, 2-amino-4-thiazolyle ou 2-(alkyle inférieur)-amino-4-thiazolyle dans lequel le groupe amino peut être protégé, et $R_b$ et $R_c$ forment ensemble un groupe syn-alcoxyimino inférieur, $R_1^b$ représente l'hydrogène, et $R_2$ représente un groupe méthoxy, alcoxy inférieur polyramifié en $\alpha$,2-halogénoalcoxy inférieur, diphénylméthoxy éventuellement substitué par un groupe alcoxy inférieur, 4-nitrobenzyloxy, tri-alkyle inférieur)-silyloxy ou 2-alcényloxy inférieur, les groupes dits »inférieurs« contenant de 1 à 7 atomes de carbone.

5. Procédé selon l'une des revendications 1 à 4, dans lequel on utilise un borohydrure de métal alcalin.

6. Procédé selon l'une des revendications 1 à 5, dans lequel on utilise le borohydrure de sodium.

7. Procédé selon l'une des revendications 1 à 6, caractérisé en ce que l'on effectue la réduction à l'aide du borohydrure de sodium en présence d'acide acétique glacial.

8. Procédé selon l'une des revendications 1 à 7, caractérisé en ce que l'on effectue la réduction à l'aide du borohydrure de sodium en présence d'aicde formique.

9. Procédé selon l'une des revendications 1 à 8, caractérisé en ce que l'on effectue la réduction à une température d'environ +15 à +20°C.

10. Procédé selon l'une des revendications 1 à 9, pour la préparation de l'ester diphénylméthylique de l'acide 7$\beta$-phénoxyacétylamino-3-hydroxycéphame-4$\alpha$-carboxylique à l'aide du borohydrure de sodium, caractérisé en ce que l'on effectue la réduction en présence d'acide acétique glacial.

11. Procédé selon l'une des revendications 1 à 9, pour la préparation de l'ester diphénylméthylique de l'acide 7$\beta$-phénoxyacétylamino-3-hydroxycéphame-4$\alpha$-carboxylique à l'aide du borohydrure de sodium, caractérisé en ce que l'on effectue la réaction en présence d'acide formique.